# EUROPEAN PATENT SPECIFICATION

(11) **EP 0 354 186 B1**
(45) Date of publication and mention of the grant of the patent: **28.08.1996**
(21) Application number: 89810578.8
(22) Date of filing: 27.07.1989
(51) Int. Cl.: A61L 2/18, A61K 9/08

(54) **A method of preserving ophthalmic solutions and compositions therefor**
Verfahren zur Konservierung ophthalmischer Lösungen und Zusammensetzungen dafür
Procédé de préservation de solutions ophtalmiques et des compositions à cet effet

(30) Priority: 04.08.1988 US 229163
(43) Date of publication of application: 07.02.1990
(60) Divisional application: 95117751.8
(73) Proprietor: CIBA-GEIGY AG, 4002 Basel (CH)
(72) Inventor: Martin, Stephen M., Roswell Georgia 30075 (US); Tsao, Fu-Pao, Lawrenceville Georgia 30243 (US)

(56) References cited:
- EP-A- 0 009 839
- EP-A- 0 265 381
- EP-A- 0 289 463
- FR-A- 2 134 666

## Description

The present invention relates to a method of preserving ophthalmic solutions with trace amounts of stabilized peroxy compounds. More particularly, this invention relates to the use of stabilized trace amounts of hydrogen peroxide as preservative in buffered saline for eye care solutions.

Hydrogen peroxide is a well-known germicidal agent. For example, hydrogen peroxide in the form of a relatively dilute solution, e.g. about 0.5 to 6 % by weight in water, is known to be effective as a disinfectant for use with contact lenses in order to kill any contaminating microorganisms.

One drawback with unstabilized dilute hydrogen peroxide solutions, however, is that without the use of a stabilizer or a combination of stabilizers, the aqueous peroxide solutions characteristically decompose over a period of time. The rate at which such dilute hydrogen peroxide solutions decompose will, of course, be dependent upon such factors as pH and the presence of trace amounts of various metal impurities, such as copper or chromium, which may act to catalytically decompose the same. Moreover, at moderately elevated temperatures, the rate of decomposition of such dilute aqueous hydrogen peroxide solutions is greatly accelerated.

A large variety of stabilizers have been proposed for use with hydrogen peroxide to deactivate trace catalytic impurities, including stannous salts, ethylene diamine tetraacetic acid, and the like. For example, U.S. Patent No. 3,860,391 discloses bleaching compositions containing hydrogen peroxide and, as a stabilizer, amino lower alkylene phosphates, including diethylene triamine penta(methylene phosphonic acid) or salts thereof, and/or hydroxy alkane phosphates, with or without additional stabilizer constituents, and adjusted to a pH of between about 9.0 and 12.0 with, e.g. sodium hydroxide, for the bleaching of cellulosic materials. Exemplified are compositions having a pH of 12.0. However, such highly basic compositions are undesirable in ophthalmically-related solutions, including eyewashes and contact lens cleaning solutions.

FR-A-2 134 666 discloses contact lens sterilizing solutions containing at least 0,025 % weight % of H₂O₂. However a bicarbonate is added to decompose the H₂O₂.

Also, British Patent No. 1,500,707 discloses a contact lens sterilizing solution using hydrogen peroxide with 200-2000 ppm of a phosphate [pyrophosphate] stabilizer at a pH of 4.5.

However, the disinfection is not carried out at a pH consistent with the ocular environment since the pH needs to be elevated to around 7. Also, the peroxide must be eliminated so as to make the solution compatible with the eye.

U.S. Patent 4,304,762 discloses stabilization of aqueous hydrogen peroxide by addition of diethylenetriamine penta(methylene phosphonic acid) or a salt thereof at a pH of about 7. However, the stabilizing phosphonate compound is reported to be particularly effective for use with alkaline hydrogen peroxide solutions. Further these solutions are disclosed as being of use as a base for liquid bleach products. There is neither any hint that trace amounts of hydrogen peroxide are effectively stabilized nor is there any indication that hydrogen peroxide might be used as a preservative for an ophthalmic solution.

Some of the eye care solutions commercially available today use benzalkonium chloride, rather than hydrogen peroxide, as a preservative. For example, contact lens solutions typically contain 0.9 % sodium chloride, buffers, surfactants, wetting agents, and 0.002 to 0.01 % benzalkonium chloride. Benzalkonium chloride is also used in other products, including isotonic decongestant ophthalmic solutions, such as Visine® eyedrops manufactured by the Leeming Division of Pfizer, Inc.

A problem exists, however, in that benzalkonium chloride, being cationic in character, reacts with proteins found in the ocular environment and causes unwanted deposits on soft contact lenses. Benzalkonium chloride and its analogs are also taken up by lens material and can have a deleterious effect on the structure of the lens [Davis, S.S. et al., "The Adsorption of Cationic Antimicrobial Agent Onto PolyHema", Colloids and Surfaces, 12, 203-212 (1984)]. In addition, benzalkonium chloride is inactivated by many compounds, including those associated with cotton and nylon fibers. Furthermore, in Swan, K.C., "Reactivity of the Ocular Tissues to Wetting Agents", Am. J. Ophthalmol., 27, 118 (1944), it was noted that repeated use of benzalkonium chloride at concentrations of 1:5000 or stronger can denature the corneal protein and cause irreversable damage. It was also found that 0.04 % to 0.05 % solutions of benzalkonium chloride can cause superficial puncture disturbance of the corneal epithelium.

Other preservatives currently in use include thimerosal, which can cause a sensitivity reaction to the eye, and sorbic acid, which commonly causes lens discoloration. The disadvantages of the commonly used preservatives such as thimerosal, benzalkonium chloride and others are discussed in the following literature: M. Sibley, et al., "Understanding Sorbic Acid-Preserved Contact Lens Solutions", International Contact lens Clinic 11 (9), 531 (1984); M. Orron, et al., "Measurement of Preservative Binding with Soflens® (polymacon) Contact lens", Aust. J. Optom., 59, 277 (1976); and M. Akers, "Considerations in selecting antimicrobial preservative agents for parenteral product development", Pharmaceutical Technology, May, p. 36 (1984).

An object of the invention is to provide a preservative for all manner of ophthalmic solutions having hydrogen peroxide compatible components which does not suffer from the aforementioned defects.

Another object of the invention is to provide preserved ophthalmic solution formulations which are free of the known art preservatives.

Yet another object of the invention is to provide a means of preserving ophthalmic solutions with hydrogen peroxide or hydrogen peroxide generating components.

Surprisingly, the disadvantages of the prior art preservatives are overcome by stabilized trace peroxy compounds provided by the present invention which may be used as a preservative in ophthalmic solutions such as eye lubrication solutions, comfort drops and ophthalmic drug formulations.

The low levels of peroxy compounds are below the commercially suitable amounts necessary for the peroxy compound to act as a disinfectant and is either low enough to be tolerable to direct application to the eye or may be made so e.g. by simple dilution with water or saline. In addition, the pH is also compatible with the ocular environment or upon the dilution indicated above is made so. For ophthalmic solutions (those which are to be instilled in the eye directly), the peroxy content and pH must per se be in the "ocular compatible range".

The invention is therefore directed to a method of preserving a ophthalmic solution as claimed in claim 1, to a preserved ophthalmic solution as claimed in claim 2, and to the use of trace amounts of hydrogen peroxide as a preservative for opthalmic solutions as claimed in claim 20.

More specifically, the invention relates to hydrogen peroxide or a source of hydrogen peroxide in trace amounts as a preservative for a ophthalmic solution, said hydrogen peroxide being especially effectively stabilized by addition of diethylene triamine penta(metyhlene phosphonic acid) or 1-hydroxyethylidene-1,1-diphosphonate, to the use of said stabilized trace amounts of hydrogen peroxide for preserving an ophthalmic solution, to ophthalmic solutions so preserved, to the manufacture of so preserved ophthalmic solutions and to a method of preserving any ophthalmic solution by adding thereto said stabilized trace amounts of stabilized hydrogen peroxide or a source of hydrogen peroxide.

For example, the trace amount of the hydrogen peroxide in these ophthalmic solutions ranges from 0.001 % (10 ppm) to 0.10 % (1000 ppm) by weight and is stabilized by 0.002 % (20 ppm) to 0.03 % (300 ppm) by weight of diethylene triamine penta(methylene phosphonic acid) or a physiologically compatible salt thereof.

Alternatively or additionally, from 0.005 % to 0.2% by weight of 1-hydroxyethylidene-1,1-diphosphonic acid and/or from 0.005 % to 0.1 % of glycerin can be added to the solution.

Trace amounts of peroxy compounds stabilized with a hydrogen peroxide stabilizer, especially diethylene triamine penta(methylene phosphonic acid) or 1-hydroxyethylidene-1,1-diphosphonic acid may be utilized as a preservative for drugs, eyewashes, or other solutions containing an active ingredient designed to be used in the ocular environment. The preservative according to the present invention may be used in any ophthalmic solution as long as the active ingredient in that solution is compatible with trace amounts of the peroxy compound Also, virtually any peroxy compound may be used so long as it is hydrolyzed in water to produce hydrogen peroxide. Examples of such sources of hydrogen peroxide, which provide an effective resultant amount of hydrogen peroxide, include sodium perborate decahydrate, sodium peroxide and urea peroxide. It has been found that peracetic acid, an organic peroxy compound, cannot be stabilized utilizing the present system.

The full scope of the present invention includes ophthalmic active agent containing solutions as well as solutions which are ophthalmic active agent free. The former group contains at least one medicinal agent for application directly to the eye. The latter group comprises such solutions as preserved lubricating solutions, among others.

It is believed that most compounds, when preserved by the present invention, are compatible with trace amounts of hydrogen peroxide. The following is a non-exhaustive, non-limiting, illustrative list of active ingredients and excipients that are compatible with the preservative according to the present invention:
atropine, homatropine, cyclopentolate, tropicamide, lachesine, dibutoline, oxyphenonium, eucatropine, ephedrine, carbachol, methacholine, pilocarpine hydrochloride, isoflurophate, physostigmine, neostigmine, lignocaine, cocaine, acetylcholine chloride, antazoline phosphate, betaxolol hydrochloride, demecarium bromide, dipivefrin hydrochloride, erythromycin, gentamicin sulfate, homatropine hydrobromide, idoxuridine, isosorbide, lanolin, naphazoline hydrochloride, neomycin sulfate, pheniramine maleate, polysorbate gelatin (Tween), pyrilamine maleate, scopolamine hydrobromide, hyaluronic acid, sodium hyaluronate, tetracaine hydrochloride, oxymetazolin, tetrahydrozoline hydrochloride, diclofenac sodium, dextran, carteolol, sulfanilamide, procaine, proparacaine hydrochloride, sulfisoxazole disolamine, indomethacin, clonidine, corynanthine, arachidonic acid, linoleic acid, H-thymidine and 3H-thymidine, inositol triphosphate, inositol phosphates, phosphatidylinositol and phosphatidylinositol phosphates.

Excipients of various types compatible with the present invention include, but are not limited to:
polysorbate gelatin (Tween), dextran, linolin, inositol phosphates, alkylsulfosuccinates, sulfosuccinamate, alkyl silicone sulfosuccinates, alkylpolyether carboxylates, alkylaryl polyethoxylamines, alkylarylsulfonates, alpha olefin sulfonates, alkyl sulfates, alkyl ether sulfates, alkanol amides and alkamides, alkylamphoterics, amphoterics based on alkyl imidazoline, betaines, alkylaminopropionates, alkyliminodipropionates, alkylamphoglycinates, alkylamphocarboxyglycinates, alkylamphocarboxypropinates, alkylamphopropionates, alkylamidopropylhydroxysultaines, alkyletherhydroxypropylsultaines, alkylamphopropylsulfonates, quaternary ammonium polymers, quaternary ammonium halides, polyacrylamide, polyacrylates, polyvinyl pyrrolidone, polyvinyl alcohol, alkylalcohol ethoxylates, hydroxyalkylcelluloses, alkylamidopropyl PG-dimoniumchloridephosphates, alkylampho PG-glycinate phosphates, glyceryl monoalkylates, sorbitan alkylates (span), pluronics, tetronics, sodium alkyl sulfates, sodium butoxyethoxy acetate, phosphate esters, glycosides, polyglycosides, mannitol, sorbitol, polyoxyethylene alkyl ethers, grillosan having the formula 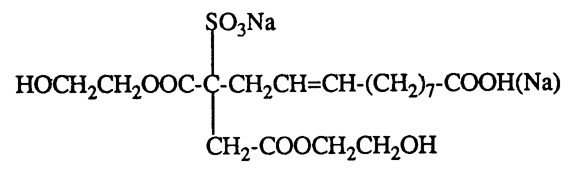 guar gum, sodium hyaluronate, polyoxyl 40 stearate or polyoxyalkylene dimethylpolysiloxane.

However, compounds having non-hindered hydroxyl groups attached to an aromatic ring, such as ketones and alcohols, or having a mercapto group, thioether, acetamido group, or aldehyde group will typically not be compatible. Such compounds believed not compatible with trace stabilized hydrogen peroxide include:
noradrenaline, adrenaline, phenylephrine hydrochloride, amethocaine, oxybuprocaine, proxymethacaine, cromolyn sodium, benoxinate hydrochloride, chloramphenicol, chlortetracycline hydrochloride, dexamethasone, dichlorphenamide, echothiophate iodide, epinephrine bitartrate, flurometholone, gramicidin, hydrocortisone, methazolamide, natamycin, prednisolone acetate, sulfacetamide (N¹-acetylsulfanilamide), tetracycline hydrochloride and timolol maleate.

The peroxy stabilizer used in the present invention may be any of the known stabilizers of peroxy compounds including phosphonates, phosphates, stannates, etc. However, physiologically compatible salts of phosphonic acids are preferred. Within this preferred group are
(a) compounds of the formula 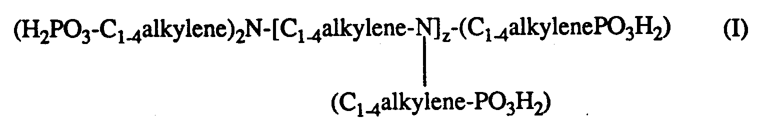 wherein z is an integer of from 0-3;
   and
(b) compounds of the formula 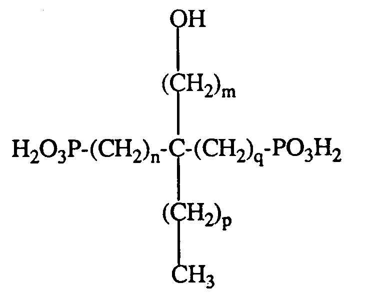 wherein each of n, m, p and q is independently 0-4,
   or physiologically compatible salts thereof. Highly preferred within formula I are compounds wherein z is 2 and compounds wherein each C₁₋₄alkylene group is C₁ or C₂. Most preferred within formula I is diethylene triamine penta(methylene-phosphonic acid, and the physiologically compatible salts thereof, marketed by Monsanto under the name Dequest® 2060. Highly preferred within formula II are compounds wherein n, m, p and q are each 0 or 1, most preferably zero, or a physiologically compatible salt thereof, marketed by Monsanto under the name Dequest® 2010.

Physiologically compatible salts of the compounds of formulae I and II include, for example, water soluble salts with conventional pharmaceutically acceptable cationic moieties, including the alkali metal, e.g. sodium, potassium, alkaline earth metal, e.g. calcium, ammonium and amine cations. Suitable amine salts include, for example, mono-, di-, and tri-lower alkyl amines, e.g. methylamine, ethylamine, diethylamine, triethylamine, dimethylamine, trimethylamine, propylamine, and the like; and mono-, di-, and tri-lower hydroxyalkyl amines, e.g. ethanolamine, diethanolamine, triethanolamine, glucamine, 2-hydroxypropylamine, and the like. By "lower" in the context of an alkyl group is meant an alkyl group having up to 6 carbon atoms, preferably up to 4 carbon atoms.

If desired, additional conventional stabilizers may be employed in conjunction with those of formulae I or II or combinations thereof in accordance with the present invention. Suitable conventional stabilizers include: water soluble stannates, such as an alkali metal or ammonium stannate, for example sodium stannate, alone or in combination with a soluble phosphate, polyphosphate or metaphosphate salt, such as an alkali metal or ammonium salt thereof; or an amino polycarboxylic acid chelating agent, such as ethylene diamine tetraacetic acid, nitrilo triacetic acid or a water soluble salt thereof, such as an alkali metal or ammonium salt, especially the sodium salt, or mixtures thereof.

Still further peroxy stabilizers which may be used in the invention include a peroxide stabilizer selected from glycerin, polyvinyl alcohol having a molecular weight in the range of about 5,000 to about 150,000 (as long as water soluble) and being at least 80 % hydrolized, propylene glycol, polyacrylic acid having a molecular weight of about 2,000 to about 100,000, diethylene glycol, and sodium hexamethaphosphate sodium polyphosphate (available from FMC under the name Hexaphos®).

The above stabilizers can be used in almost all indications previously mentioned to which the invention is applicable. However, when the solution is to come in contact with a hydrogel soft contact lens, stannate stabilizers are to be avoided as they tend to "cloud" the lens material.

Preferably, the concentration of the stabilizer of formula I or salt thereof is present in the stabilized composition in an amount between 0.006 and 0.02 % by weight of the composition, and most preferably between 0.006 and 0.0120 % by weight of the composition.

Even preferably the stabilizer of formula II is present per 100 g of solution in an amount of at least 0.024 mmole (50 ppm), preferably 0.039 mmole (80 ppm) up to 0.34 mmole (700 ppm) more preferably 0.049 mmole (100 ppm) up to 0.29 mmole (600 ppm), most preferably 0.073 mmole (150 ppm) to 0.19 mmole (400 ppm). The amounts in parentheses are for Dequest® 2010 which has a molecular weight of 206. Other stabilizers of formula II should be present in molar equivalents thereto.

The stabilizers other than those of formula I and II, if present, are employed in a physiologically tolerable amount, e.g. 20 ppm to 1000 ppm, preferably in an amount of at least 0.054 mmole (50 ppm), more preferably 0.087 mmole (80 ppm) to 1.09 mmole (1000 ppm), still more preferably from 0.109 mmole (100 ppm) to 0.87 mmole (800 ppm), most preferably 0.22 mmole (200 ppm) to 0.65 mmole.

Preferably the pH of the stabilized solution is between 5.5 and 8. More preferably, the pH of the stabilized hydrogen peroxide solution is between 6.0 and 8.0, most preferable between 6.5 and 7.5. The pH can be adjusted as desired by incorporation of suitable amounts of acid or base of a physiologically tolerable nature in the amounts employed, e.g. hydrochloric acid or e.g. sodium hydroxide.

The pH of the stabilized solution presents another advantage over the prior art since the pH's of most existing ophthalmic solutions containing hydrogen peroxide are relatively low. The pH values of available hydrogen peroxide products for contact lenses are listed as follows:

| Name of the Product | pH | % of H₂O₂ |
|---|---|---|
| AOSept (CIBA Vision) | 6.3-6.6 | 3.3-3.5 |
| Lensept (CIBA Vision) | 3.98 | 3.4 |
| Oxysept (Allergan) | 3.28 | 3.3 |
| Mirasept (Coopervision) | 3.96 | 3.6 |
| Quiksept (Bausch & Lomb) | 3.57 | 3.5 |
| Puresept (Abbott Labs) | 3.83 | 3.4 |
| Softmate II (Barnes Hind) | 3.5-3.6 | 3.5 |

Also, there may be present in the stabilized hydrogen peroxide solution according to the present invention one or more conventional, substantially inert, physiologically acceptable tonicity enhancing agents. Suitable such agents include, for example, alkali metal halides, phosphates, hydrogen phosphate, and borates. Preferred are sodium chloride, sodium phosphate monobasic and sodium phosphate dibasic. The function of such tonicity enhancing agents is to assure approximate physiologic tonicity to the solution which is instilled in the eye or to help assure such tonicity upon dilution if dilution is necessary prior to contact with the eye due to peroxide content as indicated above.

Preferably sufficient tonicity enhancing agents are present in the solution so that it is substantially isotonic or, such that, upon decomposition or dilution of the hydrogen peroxide therein, the resulting solution is substantially isotonic, e.g. substantially equivalent in tonicity to a 0.9 % by weight aqueous sodium chloride solution.

A further optional ingredient is a thickener or viscosity enhancing agent. Any of the substances known in these categories which are ocularly acceptable can be used. Typical suitable thickeners include, inter alia, polyvinylalcohol, hydroxy ethylcellulose, etc. Thickeners may be present in any amount up to an amount sufficient to raise the overall solution viscosity to about 1000 cps, preferably to not more than 100 cps.

In general, the stabilized hydrogen peroxide solutions of the present invention are characterized by their extraordinary stability, even under accelerated conditions, for example by heating the solutions to 100°C for 24 hours. Thus, the shelf life of these compositions is enhanced. Moreover, the instant compositions are characterized by their physiological tolerability subsequent to hydrogen peroxide decomposition.

Another advantage in using hydrogen peroxide in ophthalmic solutions is that the trace amount of hydrogen peroxide, especially less than 100 ppm, is destroyed once it comes in contact with the eye. For example, catalase existing in the eye tissue will cause the breakdown of the hydrogen peroxide into water and oxygen. As a result, the solution, upon application, becomes preservative free and greatly minimizes adverse reactions. The problems associated with other preservatives, such as the inability to break down innocuous compounds, are eliminated.

Formulation of the solutions of the invention can be made in any conventional manner. For example, all of the components other than the hydrogen peroxide and water can be placed in a container and fresh, preferably concentrated, hydrogen peroxide added thereto with mixing. Alternatively the dry components can be rubbed up with a small portion of liquid stabilizer, then the remainder of the stabilizer added, followed by the hydrogen peroxide, and most of the water. The viscosity enhancing agent, i.e. thickener, can then be added or the formed solution can be added to the thickener. One of ordinary skill in the art will be aware of numerous variations in the manner of formulating the solutions of the invention.

When it is desirable to "neutralize" the peroxide activity, any means known, such as rinsing, contacting the solution with a catalyst, e. g. platinum, or catalase, or any other substance known to decompose hydrogen peroxide, will suffice. Additional physiological compatible peroxide neutralizing agents including reducing agents such as pyruvic acid and suitable salts thereof such as the sodium salt.

The following examples are presented for illustrative purposes and are not intended to limit the scope of this invention, but to demonstrate the stability of the peroxy solutions as stabilized in accordance with the present invention. All parts are by weight unless otherwise indicated.

Example 1: Dissolve 0.610 g of sodium chloride, 0.005 g of sodium tetraborate·10 H₂O, 0.5 g of boric acid, 0.006 g of diethylene triamine penta(methylene phosphonic acid) and 0.1 g of tetrahydrozoline hydrochloride in 80 ml of purified deionized water. Add 0.0225 g of sodium perborate, add water up to 100 ml and adjust the pH to 7 by the addition dropwise of dilute hydrochloric acid or sodium hydroxide. The resulting solution is then heated to 100°C for a period of 24 hours. The solution possesses a "hot stability" of above 90 % which is the ratio of the hydrogen peroxide present in the after-heated sample to the before-heated sample, multiplied by 100 [%].

Example 2: The procedure of example 1 is duplicated, except that 0.1 g of sodium [o-[(2,6-dichlorophenyl)amino]phenyl]acetate replace the tetrahydrozoline hydrochloride. The hot stability is found to be 91.0 %.

Example 3: The procedure of example 1 is duplicated, except that 0.5 g of pilocarpine hydrochloride replace the tetrahydrozoline hydrochloride. The hot stability is found to be 74.3 %.

Example 4: The procedure of example 1 is duplicated, except that 0.1 g of naphazoline hydrochloride replace the tetrahydrozoline hydrochloride. The hot stability is found to be 81.3 %.

Example 5: Dissolve 0.61 g of sodium chloride, 0.50 g boric acid, 0.005 g of sodium borate decahydrate, 0.05 g Grillosan (from RITA), and 0.006 g diethylene triamine penta(methylene phosphonic acid) in 80 ml of deionized water. Add 0.0238 g sodium perborate. Add water up to 100 ml and adjust the pH to 7 by the addition dropwise of diluted hydrochloric acid or sodium hydroxide. The resulting solution is then heated to 100°C for 24 hours. The hot stability of this solution is 93.1 %.

Example 6: The procedure of example 5 is duplicated except that the 0.05 g Grillosan are replaced by 0.5 g propylene glycol. The hot stability of this solution is above 92 %.

Example 7: The procedure of example 5 is duplicated except that the 0.05 g Grillosan are replaced by 0.05 g glycerin and an additional 0.01 g magnesium chloride. The hot stability of this solution is above 94 %.

Example 8: The procedure of example 5 is duplicated except that the 0.05 g Grillosan are replaced by 0.1 g magnesium chloride. The hot stability of this solution is above 96 %.

Example 9: The procedure of example 5 is duplicated except that the 0.05 g Grillosan are replaced by 0.5 g SIPEX EST-30 (sodium trideceth sulfate, CAS No. 68585-34-2, from Alcolac). The hot stability of this solution is above 92 %.

Example 10: The procedure of example 5 is duplicated except that the 0.05 g Grillosan are replaced by 0.5 g Pluronic F-127 (BASF). The hot stability of this solution is above 93 %.

Example 11: Dissolve 0.35 g of sodium chloride, 0.35 g of potassium chloride, 0.58 g boric acid, 0.005 g sodium borate decahydrate and 0.006 g diethylene triamine penta(methylene phosphonic acid) in 80 ml of deionized water. Add 0.0238 g sodium perborate. Add water up to 100 ml and adjust the pH to 7 by the addition dropwise of diluted hydrochloric acid or sodium hydroxide. The hot stability of this solution is above 91 %.

Example 12: The procedure of example 11 is duplicated except that the 0.35 g of potassium chloride are replaced by 0.35 g of calcium chloride. The hot stability of this solution is above 93 %.

Example 13: The procedure of example 6 is duplicated except that the 0.5 g propylene glycol and 0.0238 g sodium perborate are replaced by 1 g propylene glycol and 0.0476 g sodium perborate. The hot stability of this solution is above 98 %.

Example 14: The procedure of example 13 is duplicated except that the 1 g propylene glycol is replaced by 0.1 g of citric acid. The hot stability of this solution is above 94 %.

Example 15: Dissolve 0.61 g of sodium chloride, 0.50 g boric acid, 0.005 g sodium borate decahydrate and 0.006 g diethylene triamine penta(methylene phosphonic acid) in 80 ml of deionized water. Add 0.0133 g sodium peroxide (from Mallinckrodt Cat. No. 7864). Add water up to 100 ml and adjust the pH to 7 by the addition dropwise of diluted hydrochloric acid or sodium hydroxide. The hot stability of this solution is above 93 %.

Example 16: The procedure of example 15 is duplicated except that the 0.0133 g sodium peroxide are replaced by 0.0138 g of urea hydrogen peroxide (from Aldrich Cat. No. 28913-2). The hot stability of this solution is above 53.6 %.

Example 17: Dissolve 0.61 g of sodium chloride, 0.50 g boric acid, 0.005 g sodium borate decahydrate, 0.1 g sulfanilamide (Sigma Cat. No. S-9251) and 0.006 g diethylene triamine penta(methylene phosphonic acid) in 80 ml of deionized water. Add 0.0238 g sodium perborate. Add water up to 100 ml and adjust the pH to 7 by the addition dropwise of diluted hydrochloric acid or sodium hydroxide. The hot stability of this solution is above 92 %.

Example 18: Dissolve 0.61 g of sodium chloride, 0.50 g boric acid, 0.005 g sodium borate decahydrate, and various amounts of 1-hydroxyethylidene-1,1-diphosphonic acid in 80 ml of deionized water. Add 0.0238 g sodium perborate. Add water up to 100 ml and adjust the pH to 7 by the addition dropwise of diluted hydrochloric acid or sodium hydroxide. The hot stabilities of these solutions are listed as follows:

| 1-Hydroxyethylidene-1,1-diphosphonic acid (g) | Hot Stability % |
|---|---|
| 0.03 | 87.1 |
| 0.05 | 90.3 |
| 0.08 | 96.8 |

Example 19: This is an example of 1-hydroxyethylidene-1,1-diphosphonic acid as a stabilizer: The procedure of example 18 is duplicated except that various amounts of glycerin are added. The hot stabilities of these solutions are listed as follows:

| 1-Hydroxyethylidene-1,1-diphosphonic acid (g) | Glycerin (g) | Hot Stability % |
|---|---|---|
| 0.03 | 0.03 | 90.3 |
| 0.05 | 0.03 | 93.5 |
| 0.08 | 0.03 | 93.5 |
| 0.03 | 0.05 | 90.3 |
| 0.05 | 0.05 | 90.3 |
| 0.08 | 0.05 | 93.5 |

Example 20: The procedure of example 17 is duplicated except that the 0.1 g sulfanilamide are replaced by 0.1 g hyaluronic acid (from Streptococcus Zooepidermicus). The hot stability of this solution is about 86 %.

Example 21: The procedure of example 20 is duplicated except that the 0.1 g hyaluronic acid (from Streptococcus Zooepidermicus) are replaced by 0.1 g hyaluronic acid (from Rooster Comb.). The hot stability of this solution is above 96 %.

Example 22: The procedure of example 20 is duplicated except that the 0.1 g hyaluronic acid are replaced by 0.1 g sodium hyaluronate. The hot stability of this solution is above 92 %.

Example 23: The procedure of example 22 is duplicated except that the 0.1 g sodium hyaluronate are replaced by 0.025 g oxymetazoline. The hot stability of this solution is above 87 %.

Example 24: Microbial preservative effectiveness test results of 50 ppm hydrogen peroxide in a borate buffer are listed as follows:

### PRESERVATIVE EFFECTIVENESS TEST (50 ppm H₂O₂ solution)

| Challenge Microbe | Original Inoculum | Day 7 | Day 14 | Rechallenge on Day 14 with Inoculum |
|---|---|---|---|---|
| E. coli | 1.67x10⁶ | Zero | Zero | 1.60x10⁵ |
| P. aeruginosa | 2.04x10⁶ | Zero | Zero | 1.94x10⁵ |
| S. aureus | 1.09x10⁶ | 1.75x10² | Zero | 1.74x10⁵ |
| A. niger | 1.46x10⁵ | 2.15x10⁴ | 8.40x10³ | 1.00x10⁵ |
| C. albicans No. 1 | 1.63x10⁶ | 8.00x10² | 2.40x10² | 1.41x10⁵ |
| C. albicans No. 2 | 1.63x10⁶ | 1.35x10³ | 3.40x10² | 1.41x10⁵ |
| C. albicans No. 3 | 1.63x10⁶ | 1.75x10³ | 4.90x10² | 1.41x10⁵ |

| Challenge Microbe | Day 21 | Day 28 | % Reduction | Pass/Fail |
|---|---|---|---|---|
| E. coli | Zero | Zero | 100 % | Pass |
| P. aeruginosa | Zero | Zero | 100 % | Pass |
| S. aureus | Zero | Zero | 100 % | Pass |
| A. niger | 4.65x10⁴ | 4.50x10⁴ | | Pass |
| C. albicans No. 1 | 1.09x10⁵ | 3.90x10⁴ | | Pass |
| C. albicans No. 2 | 1.12x10⁵ | 3.55x10⁴ | | Pass |
| C. albicans No. 3 | 1.26x10⁵ | 3.40x10⁴ | | Pass |

## Claims (Claims for the following Contracting State(s): AT, BE, CH, DE, FR, GB, IT, LI, LU, NL, SE)

1. A method of preserving an ophthalmic solution comprising an ophthalmic active ingredient or excipient which is compatible with hydrogen peroxide and which solution is intended for being instilled directly in the eye of a human, said solution being substantially isotonic and having an ocularly compatible pH, comprising the steps of:
a) adding to said solution a source of hydrogen peroxide, with the exception of peracetic acid, for providing an effective trace amount of resultant hydrogen peroxide to achieve a desired germicidal capacity, and
b) dissolving in said solution an effective amount of at least one hydrogen peroxide stabilizer,
wherein said resultant amount of hydrogen peroxide is from 0.001 % to 0.10 % by weight, provided that said solution, if having a pH in excess of 8, does not comprise sodium bicarbonate in an amount sufficient to decompose hydrogen peroxide.

2. A preserved ophthalmic solution comprising an ophthalmic active ingredient or excipient which is compatible with hydrogen peroxide and which solution is intended for being instilled directly in the eye of a human, said solution being substantially isotonic and having an ocularly compatible pH, effectively preserved by
a) having added to said solution a source of hydrogen peroxide, with the exception of peracetic acid, for providing an effective trace amount of resultant hydrogen peroxide to achieve a desired germicidal capacity, and
b) having dissolved in said solution an effective amount of at least one hydrogen peroxide stabilizer,
wherein said resultant amount of hydrogen peroxide is from 0.001 % to 0.10 % by weight, provided that said solution, if having a pH in excess of 8, does not comprise sodium bicarbonate in an amount sufficient to decompose hydrogen peroxide.

3. The method of claim 1, wherein said trace amount of hydrogen peroxide is the only preservative used.

4. The method of claim 1, wherein said hydrogen peroxide source is selected from the group consisting of hydrogen peroxide, sodium perborate decahydrate, sodium peroxide and urea peroxide.

5. The method of claim 1, wherein said stabilizer is diethylene triamine penta(methylene phosphonic acid) or 1-hydroxyethylidene-1,1-diphosphonic acid or a physiologically compatible salt thereof.

6. The method of claim 5, wherein said effective amount of diethylene triamine penta(methylene phosphonic acid), or a physiologically compatible salt thereof, is from 0.002 % to 0.03 % by weight and said effective amount of 1-hydroxyethylidene-1,1-diphosphonic acid or physiologically compatible salt thereof is from 0.005 % to 0.2 % by weight.

7. The method of claim 6, wherein said stabilizer is diethylene triamine penta(methylene phosphonic acid).

8. The method of claim 6, wherein said stabilizer is 1-hydroxyethylidene-1,1-diphosphonic acid.

9. The method of claim 8, and further comprising the step of adding an effective amount of glycerin.

10. The method of claim 9, wherein said glycerin is added in an amount from 0.003 % to 0.1% by weight.

11. The method of claim 1, wherein said resultant amount of hydrogen peroxide is from 0.001 % to 0.10 % by weight and said effective amount of diethylene triamine penta(methylene phosphonic acid), or a physiologically compatible salt thereof, is from 0.002% to 0.03 % by weight.

12. The method of claim 11, and further comprising the step of adding an effective amount of 1-hydroxyethylidane-1,1-diphosphonic acid as a hydrogen peroxide stabilizer.

13. The method of claim 12, and further comprising the step of adding an effective amount of glycerin.

14. The method of claim 13, wherein said glycerin is added in an amount from 0.005 % to 0.1 % by weight.

15. The method of claim 1, and further comprising the step of adding a tonicity agent.

16. The method of one or more of claims 1 to 15 further comprising the step of adjusting the pH of said solution from 5.5 to 8.

17. The method of claim 16 and further comprising the step of adding an ophthalmically acceptable active ingredient to said solution which is compatible with said trace amounts of hydrogen peroxide.

18. The method according to claim 16, and further comprising the step of adding to said solution a buffer and saline in an amount so as to render the solution substantially isotonic.

19. The method of claim 18 wherein the solution has a tonicity of 0.9 % sodium chloride.

20. The use of trace amounts of a source of hydrogen peroxide, with the exception of peracetic acid, the resulting hydrogen peroxide being stabilized by an effective amount of a hydrogen peroxide stabilizer, as a preservative for an ophthalmic solution comprising an ophthalmic active ingredient or excipient which is compatible with hydrogen peroxide and which solution is being intended for being instilled directly in the eye of a human, said solution being substantially isotonic and having an ocularly compatible pH, wherein said resultant amount of hydrogen peroxide is from 0.001 % to 0.10 % by weight,
provided that said solution, if having a pH in excess of 8, does not comprise sodium bicarbonate in an amount sufficient to decompose hydrogen peroxide.

## Claims (Claims for the following Contracting State(s): ES, GR)

1. A method of preserving an ophthalmic solution comprising an ophthalmic active ingredient or excipient which is compatible with hydrogen peroxide and which solution is intended for being instilled directly in the eye of a human, said solution being substantially isotonic and having an ocularly compatible pH, comprising the steps of:
a) adding to said solution a source of hydrogen peroxide, with the exception of peracetic acid, for providing an effective trace amount of resultant hydrogen peroxide to achieve a desired germicidal capacity, and
b) dissolving in said solution an effective amount of at least one hydrogen peroxide stabilizer,
wherein said resultant amount of hydrogen peroxide is from 0.001 % to 0.10 % by weight, provided that said solution, if having a pH in excess of 8, does not comprise sodium bicarbonate in an amount sufficient to decompose hydrogen peroxide.

2. A process for the manufacture of an effectively preserved ophthalmic solution comprising an ophthalmic active ingredient or excipient which is compatible with hydrogen peroxide and which solution is intended for being instilled directly in the eye of a human, said solution being substantially isotonic and having an ocularly compatible pH, comprising the steps of
a) adding to said solution a source of hydrogen peroxide, with the exception of peracetic acid, for providing an effective trace amount of resultant hydrogen peroxide to achieve a desired germicidal capacity, and
b) dissolving in said solution an effective amount of at least one hydrogen peroxide stabilizer,
wherein said resultant amount of hydrogen peroxide is from 0.001 % to 0.10 % by weight, provided that said solution, if having a pH in excess of 8, does not comprise sodium bicarbonate in an amount sufficient to decompose hydrogen peroxide.

3. The method of claim 1, wherein said trace amount of hydrogen peroxide is the only preservative used.

4. The method of claim 1, wherein said hydrogen peroxide source is selected from the group consisting of hydrogen peroxide, sodium perborate decahydrate, sodium peroxide and urea peroxide.

5. The method of claim 1, wherein said stabilizer is diethylene triamine penta(methylene phosphonic acid) or 1-hydroxyethylidene- 1,1-diphosphonic acid or a physiologically compatible salt thereof.

6. The method of claim 5, wherein said effective amount of diethylene triamine penta(methylene phosphonic acid), or a physiologically compatible salt thereof, is from 0.002 % to 0.03 % by weight and said effective amount of 1-hydroxyethylidene-1,1-diphosphonic acid or physiologically compatible salt thereof is from 0.005 % to 0.2 % by weight.

7. The method of claim 6, wherein said stabilizer is diethylene triamine penta(methylene phosphonic acid).

8. The method of claim 6, wherein said stabilizer is 1 hydroxyethylidene-1,1-diphosphonic acid.

9. The method of claim 8, and further comprising the step of adding an effective amount of glycerin.

10. The method of claim 9, wherein said glycerin is added in an amount from 0.003 % to 0.1 % by weight.

11. The method of claim 1, wherein said resultant amount of hydrogen peroxide is from 0.001 % to 0.10 % by weight and said effective amount of diethylene triamine penta(methylene phosphonic acid), or a physiologically compatible salt thereof, is from 0.002 % to 0.03 % by weight.

12. The method of claim 11, and further comprising the step of adding an effective amount of 1-hydroxyethylidene-1,1-diphosphonic acid as a hydrogen peroxide stabilizer.

13. The method of claim 12, and further comprising the step of adding an effective amount of glycerin.

14. The method of claim 13, wherein said glycerin is added in an amount from 0.005 % to 0.1 % by weight.

15. The method of claim 1, and further comprising the step of adding a tonicity agent.

16. The method of one or more of claims 1 to 15 further comprising the step of adjusting the pH of said solution from 5.5 to 8.

17. The method of claim 16 and further comprising the step of adding an ophthalmically acceptable active ingredient to said solution which is compatible with said trace amounts of hydrogen peroxide.

18. The method according to claim 16, and further comprising the step of adding to said solution a buffer and saline in an amount so as to render the solution substantially isotonic.

19. The method of claim 18 wherein the solution has a tonicity of 0.9 % sodium chloride.

20. The use of trace amounts of a source of hydrogen peroxide, with the exception of peracetic acid, the resulting hydrogen peroxide being stabilized by an effective amount of a hydrogen peroxide stabilizer, as a preservative for an ophthalmic solution comprising an ophthalmic active ingredient or excipient which is compatible with hydrogen peroxide and which solution is being intended for being instilled directly in the eye of a human, said solution being substantially isotonic and having an ocularly compatible pH, wherein said resultant amount of hydrogen peroxide is from 0.001 % to 0.10 % by weight,
provided that said solution, if having a pH in excess of 8, does not comprise sodium bicarbonate in an amount sufficient to decompose hydrogen peroxide.

## Patentansprüche (Patentansprüche für folgende(n) Vertragsstaat(en): AT, BE, CH, DE, FR, GB, IT, LI, LU, NL, SE)

1. Verfahren zur Konservierung einer ophthalmischen Lösung, die einen ophthalmisch aktiven Inhaltsstoff oder Arzneimittelträger enthält, der mit Wasserstoffperoxid verträglich ist, und die direkt ins Auge eines Menschen geträufelt werden soll, wobei die Lösung im wesentlichen isotonisch ist und einen Augen-verträglichen pH-Wert aufweist, wobei das Verfahren die folgenden Schritte umfaßt:
a) der Lösung eine Wasserstoffperoxid-Quelle zuzusetzen, ausgenommen Peressigsäure, um eine wirksame Spurenmenge an so erhaltenem Wasserstoffperoxid zur Verfügung zu stellen, um eine gewünschte keimtötende Fähigkeit zu erreichen, und
b) in der Lösung eine wirksame Menge von mindestens einem Wasserstoffperoxid Stabilisator zu lösen,
wobei die so erhaltene Menge an Wasserstoffperoxid 0,001 - 0,10 Gew.-% beträgt, mit der Maßgabe, daß die Lösung, wenn sie einen pH-Wert von mehr als 8 aufweist, Natriumbicarbonat nicht in einer Menge enthält, die ausreichend ist, um Wasserstoffperoxid zu zersetzen.

2. Konservierte, ophthalmische Lösung, die einen ophthalmisch aktiven Inhaltsstoff oder Arzneimittelträger enthält, der mit Wasserstoffperoxid verträglich ist, und die direkt ins Auge eines Menschen geträufelt werden soll, wobei die Lösung im wesentlichen isotonisch ist und einen Augen-verträglichen pH aufweist, dadurch wirksam konserviert, daß
a) der Lösung eine Wasserstoffperoxid-Quelle zugesetzt wurde, ausgenommen Peressigsäure, um eine wirksame Spurenmenge an so erhaltenem Wasserstoffperoxid zur Verfügung zu stellen, um eine gewünschte keimtötende Fähigkeit zu erreichen, und
b) in der Lösung eine wirksame Menge von mindestens einem Wasserstoffperoxid-Stabilisator gelöst wurde,
wobei die so erhaltene Menge an Wasserstoffperoxid 0,001 - 0,10 Gew.-% beträgt, mit der Maßgabe, daß die Lösung, wenn sie einen pH-Wert von mehr als 8 aufweist, nicht Natriumbicarbonat in einer Menge enthält, die ausreichend ist, um Wasserstoffperoxid zu zersetzen.

3. Verfahren nach Anspruch 1, wobei die Spurenmenge an Wasserstoffperoxid das einzige verwendete Konservierungsmittel ist.

4. Verfahren nach Anspruch 1, wobei die Wasserstoffperoxid-Quelle ausgewählt ist aus der Gruppe bestehend aus Wasserstoffperoxid, Natriumperborat-Decahydrat, Natriumperoxid und Harnstoffperoxid.

5. Verfahren nach Anspruch 1, wobei der Stabilisator Diethylentriaminpentamethylenphosphonsäure oder 1-Hydroxyethyliden-1,1-diphosphonsäure oder ein physiologisch verträgliches Salz davon ist.

6. Verfahren nach Anspruch 5, wobei die wirksame Menge an Diethylentriaminpentamethylenphosphonsäure oder einem physiologisch verträglichen Salz davon 0,002 - 0,03 Gew.-% beträgt und die wirksame Menge an 1-Hydroxyethyliden-1,1-diphosphonsäure oder einem physiologisch verträglichen Salz davon 0,005 - 0,2 Gew.-% beträgt.

7. Verfahren nach Anspruch 6, wobei der Stabilisator Diethylentriaminpentamethylenphosphonsäure ist.

8. Verfahren nach Anspruch 6, wobei der Stabilisator 1-Hydroxyethyliden-1,1-diphosphonsäure ist.

9. Verfahren nach Anspruch 8, das weiter den Schritt der Zugabe einer wirksamen Menge an Glycerin umfaßt.

10. Verfahren nach Anspruch 9, wobei das Glycerin in einer Menge von 0,003 - 0,1 Gew.-% zugegeben wird.

11. Verfahren nach Anspruch 1, wobei die so erhaltene Menge Wasserstoffperoxid 0,001 - 0,10 Gew.-% und die wirksame Menge an Diethylentriaminpentamethylenphosphonsäure oder einem physiologisch verträglichen Salz davon 0,002 - 0,03 Gew.-% beträgt.

12. Verfahren nach Anspruch 11, das weiter den Schritt der Zugabe einer wirksamen Menge 1-Hydroxyethyliden-1,1-diphosphonsäure als Wasserstoffperoxid-Stabilisator umfaßt.

13. Verfahren nach Anspruch 12, das weiter den Schritt der Zugabe einer wirksamen Menge Glycerin umfaßt.

14. Verfahren nach Anspruch 13, wobei das Glycerin in einer Menge von 0,005 - 0,1 Gew.-% zugegeben wird.

15. Verfahren nach Anspruch 1, das weiter den Schritt der Zugabe eines Tonizitätsmittels umfaßt.

16. Verfahren nach einem oder mehreren der Ansprüche 1 bis 15, das weiter den Schritt der Einstellung des pH-Wertes der Lösung auf 5,5 bis 8 umfaßt.

17. Verfahren nach Anspruch 16, das weiter den Schritt der Zugabe eines ophthalmisch verträglichen, aktiven Inhaltsstoffs zu der Lösung umfaßt, der mit den Spurenmengen Wasserstoffperoxid verträglich ist.

18. Verfahren nach Anspruch 16, das weiter den Schritt der Zugabe eines Puffers und einer Salzlösung zu der Lösung in einer Menge umfaßt, die die Lösung im wesentlichen isotonisch macht.

19. Verfahren nach Anspruch 18, wobei die Lösung eine Tonizität von 0,9 % Natriumchlorid aufweist.

20. Verwendung von Spurenmengen einer Wasserstoffperoxid-Quelle, ausgenommen Peressigsäure, wobei das so erhaltene Wasserstoffperoxid durch eine wirksame Menge eines Wasserstoffperoxid-Stabilisators stabilisiert wird, als Konservierungsmittel für eine ophthalmische Lösung, die einen ophthalmisch aktiven Inhaltsstoff oder Arzneimittelträger enthält, der mit Wasserstoffperoxid verträglich ist, und die direkt ins Auge eines Menschen geträufelt werden soll, wobei die Lösung im wesentlichen isotonisch ist und einen Augen-verträglichen pH-Wert aufweist, wobei die so erhaltene Menge Wasserstoffperoxid 0,001 - 0,10 Gew.-% beträgt, mit der Maßgabe, daß die Lösung, wenn sie einen pH-Wert von mehr als 8 aufweist, Natriumbicarbonat nicht in einer Menge enthält, die ausreichend ist, um Wasserstoffperoxid zu zersetzen.

## Patentansprüche (Patentansprüche für folgende(n) Vertragsstaat(en): ES, GR)

1. Verfahren zur Konservierung einer ophthalmischen Lösung, die einen ophthalmisch aktiven Inhaltsstoff oder Arzneimittelträger enthält, der mit Wasserstoffperoxid verträglich ist, und die direkt ins Auge eines Menschen geträufelt werden soll, wobei die Lösung im wesentlichen isotonisch ist und einen Augen-verträglichen pH-Wert aufweist, wobei das Verfahren die folgenden Schritte umfaßt:
a) der Lösung eine Wasserstoffperoxid-Quelle zuzusetzen, ausgenommen Peressigsäure, um eine wirksame Spurenmenge an so erhaltenem Wasserstoffperoxid zur Verfügung zu stellen, um eine gewünschte keimtötende Fähigkeit zu erreichen, und
b) in der Lösung eine wirksame Menge von mindestens einem Wasserstoffperoxid-Stabilisator zu lösen,
wobei die so erhaltene Menge Wasserstoffperoxid 0,001 - 0,10 Gew.-% beträgt, mit der Maßgabe, daß die Lösung, wenn sie einen pH-Wert von mehr als 8 aufweist, Natriumbicarbonat nicht in einer Menge enthält, die ausreichend ist, um Wasserstoffperoxid zu zersetzen.

2. Verfahren zur Herstellung einer wirksam konservierten, ophthalmischen Lösung, die einen ophthalmisch aktiven Inhaltsstoff oder Arzneimittelträger enthält, der mit Wasserstoffperoxid verträglich ist, und die direkt ins Auge eines Menschen geträufelt werden soll, wobei die Lösung im wesentlichen isotonisch ist und einen Augen-verträglichen pH-Wert aufweist, wobei das Verfahren die folgenden Schritte umfaßt:
a) der Lösung eine Wasserstoffperoxid-Quelle zuzusetzen, ausgenommen Peressigsäure, um eine wirksame Spurenmenge an so erhaltenem Wasserstoffperoxid zur Verfügung zu stellen, um eine gewünschte keimtötende Fähigkeit zu erreichen, und
b) in der Lösung eine wirksame Menge von mindestens einem Wasserstoffperoxid-Stabilisator zu lösen,
wobei die so erhaltene Menge Wasserstoffperoxid 0,001 - 0,10 Gew.-% beträgt, mit der Maßgabe, daß die Lösung, wenn sie einen pH-Wert von mehr als 8 aufweist, Natriumbicarbonat nicht in einer Menge enthält, die ausreichend ist, um Wasserstoffperoxid zu zersetzen.

3. Verfahren nach Anspruch 1, wobei die Spurenmenge an Wasserstoffperoxid das einzige verwendete Konservierungsmittel ist.

4. Verfahren nach Anspruch 1, wobei die Wasserstoffperoxid-Quelle ausgewählt ist aus der Gruppe bestehend aus Wasserstoffperoxid, Natriumperborat-Decahydrat, Natriumperoxid und Harnstoffperoxid.

5. Verfahren nach Anspruch 1, wobei der Stabilisator Diethylentriaminpentamethylenphosphonsäure oder 1-Hydroxyethyliden-1,1-diphosphonsäure oder ein physiologisch verträgliches Salz davon ist.

6. Verfahren nach Anspruch 5, wobei die wirksame Menge an Diethylentriaminpentamethylenphosphonsäure oder einem physiologisch verträglichen Salz davon 0,002 - 0,03 Gew.-% beträgt und die wirksame Menge an 1-Hydroxyethyliden-1,1-diphosphonsäure oder einem physiologisch verträglichen Salz davon 0,005 - 0,2 Gew.-% beträgt.

7. Verfahren nach Anspruch 6, wobei der Stabilisator Diethylentriaminpentamethylenphosphonsäure ist.

8. Verfahren nach Anspruch 6, wobei der Stabilisator 1-Hydroxyethyliden-1,1-diphosphonsäure ist.

9. Verfahren nach Anspruch 8, das weiter den Schritt der Zugabe einer wirksamen Menge Glycerin umfaßt.

10. Verfahren nach Anspruch 9, wobei das Glycerin in einer Menge von 0,003 - 0,1 Gew.-% zugegeben wird.

11. Verfahren nach Anspruch 1, wobei die so erhaltene Menge Wasserstoffperoxid 0,001 - 0,10 Gew.-%, und die wirksame Menge Diethylentriaminpentamethylenphosphonsäure oder einem physiologisch verträglichen Salz davon 0,002 - 0,03 Gew.-% beträgt.

12. Verfahren nach Anspruch 11, das weiter den Schritt der Zugabe einer wirksamen Menge 1-Hydroxyethyliden-1,1-diphosphonsäure als Wasserstoffperoxid-Stabilisator umfaßt.

13. Verfahren nach Anspruch 12, das weiter den Schritt der Zugabe einer wirksamen Menge Glycerin umfaßt.

14. Verfahren nach Anspruch 13, wobei das Glycerin in einer Menge von 0,005 - 0,1 Gew.-% zugegeben wird.

15. Verfahren nach Anspruch 1, das weiter der Schritt der Zugabe eines Tonizitätsmittels umfaßt.

16. Verfahren nach einem der Ansprüche 1 bis 15, das weiter den Schritt der Einstellung des pH-Wertes der Lösung auf 5,5 bis 8 umfaßt.

17. Verfahren nach Anspruch 16, das weiter den Schritt der Zugabe eines ophthalmisch verträglichen, aktiven Inhaltsstoffs zu der Lösung umfaßt, der mit den Spurenmengen Wasserstoffperoxid verträglich ist.

18. Verfahren nach Anspruch 16, das weiter den Schritt der Zugabe eines Puffers und einer Salzlösung zu der Lösung in einer Menge umfaßt, die die Lösung im wesentlichen isotonisch macht.

19. Verfahren nach Anspruch 18, wobei die Lösung eine Tonizität von 0,9 % Natriumchlorid aufweist.

20. Verwendung von Spurenmengen einer Wasserstoffperoxid-Quelle, ausgenommen Peressigsäure, wobei das so erhaltene Wasserstoffperoxid durch eine wirksame Menge eines Wasserstoffperoxid-Stabilisators stabilisiert wird, als Konservierungsmittel für eine ophthalmische Lösung, die einen ophthalmisch aktiven Inhaltsstoff oder Arzneimittelträger enthält, der mit Wasserstoffperoxid verträglich ist, und die direkt ins Auge eines Menschen geträufelt werden soll, wobei die Lösung im wesentlichen isotonisch ist und einen Augen-verträglichen pH-Wert aufweist, wobei die so erhaltene Menge Wasserstoffperoxid 0,001 - 0,10 Gew.-% beträgt, mit der Maßgabe, daß die Lösung, wenn sie einen pH-Wert von mehr als 8 aufweist, Natriumbicarbonat nicht in einer Menge enthält, die ausreichend ist, um Wasserstoffperoxid zu zersetzen.

## Revendications (Revendications pour l'(les) Etat(s) contractant(s) suivant(s): AT, BE, CH, DE, FR, GB, IT, LI, LU, NL, SE)

1. Procédé de conservation d'une solution ophtalmique comprenant un principe actif ophtalmique ou un excipient, qui est compatible avec le peroxyde d'hydrogène, cette solution étant conçue pour être instillée directement dans l'oeil d'un être humain, ladite solution étant sensiblement isotonique et ayant un pH oculairement compatible, comprenant les étapes de:
a) addition à ladite solution d'une source de peroxyde d'hydrogène, à l'exception de l'acide péracétique, pour fournir une quantité efficace de peroxyde d'hydrogène résultant afin d'obtenir la capacité germicide désirée, et
b) dissolution dans ladite solution d'une quantité efficace d'au moins un stabilisateur de peroxyde d'hydrogène,
ladite quantité résultante de peroxyde d'hydrogène étant de 0,001% à 0,10% en poids, à condition que ladite solution, si elle a un pH dépassant 8, ne comprend pas de bicarbonate de sodium en une quantité suffisante pour décomposer le peroxyde d'hydrogène.

2. Solution ophtalmique conservée comprenant un principe actif ophtalmique ou excipient qui est compatible avec le peroxyde d'hydrogène, cette solution étant conçue pour être instillée directement dans l'oeil d'un être humain, ladite solution étant sensiblement isotonique et ayant un pH oculairement compatible, efficacement conservée par
a) le fait qu'on a ajouté à ladite solution une source de peroxyde d'hydrogène, à l'exception de l'acide péracétique, pour fournir une trace efficace de peroxyde d'hydrogène résultant afin d'obtenir la capacité germicide désirée, et
b) le fait qu'on a dissous dans ladite solution une quantité efficace d'au moins un stabilisateur de peroxyde d'hydrogène,
ladite quantité résultante de peroxyde d'hydrogène étant de 0,001% à 0,10% en poids, à condition que ladite solution si elle a un pH de 8, ne comprend pas de bicarbonate de sodium en une quantité suffisante pour décomposer le peroxyde d'hydrogène.

3. Procédé de la revendication 1, dans lequel ladite trace de peroxyde d'hydrogène est le seul agent de conservation utilisé.

4. Procédé de la revendication 1, dans lequel ladite source de peroxyde d'hydrogène est choisie dans le groupe constitué par le peroxyde d'hydrogène, le perborate de sodium décahydraté, le peroxyde de sodium et le peroxyde durée.

5. Procédé de la revendication 1, dans lequel ledit stabilisateur d'acide diéthylène triamine penta(méthylène phosphonique) ou l'acide 1-hydroxyéthylidène-1,1-diphosphonique ou un de leurs sels physiologiquement compatibles.

6. Procédé de la revendication 5, dans lequel ladite quantité efficace d'acide diéthylène triamine penta(méthylène phosphonique) ou d'un de ses sels physiologiquement compatibles est de 0,002% à 0,03% en poids et ladite quantité efficace d'acide 1-hydroxyéthylidène-1,1-diphosphonique ou d'un de ses sels physiologiquement compatibles est de 0,005% à 0,2% en poids.

7. Procédé de la revendication 6, dans lequel ledit stabilisateur est l'acide diéthylène triamine penta(méthylène phosphonique).

8. Procédé de la revendication 6, dans lequel ledit stabilisateur est l'acide 1-hydroxyéthylidène-1,1-diphosphonique.

9. Procédé de la revendication 8, comprenant en outre l'étape d'addition d'une quantité efficace de glycérine.

10. Procédé de la revendication 9, dans lequel ladite glycérine est ajoutée en une quantité de 0,003% à 0,1% en poids.

11. Procédé de la revendication 1, dans lequel ladite quantité résultante de peroxyde d'hydrogène est de 0,001% à 0,10% en poids et ladite quantité efficace d'acide diéthylène triamine penta(méthylène phosphonique) ou d'un de ses sels physiologiquement compatibles, est de 0,002% à 0,03% en poids.

12. Procédé de la revendication 11, comprenant en outre l'étape d'addition d'une quantité efficace d'acide 1-hydroxyéthylidène-1,1-diphosphonique comme stabilisateur de peroxyde d'hydrogène.

13. Procédé de la revendication 12, comprenant en outre l'étape d'addition d'une quantité efficace de glycérine.

14. Procédé de la revendication 13, dans lequel ladite glycérine est ajoutée en une quantité de 0,005% à 0,1% en poids.

15. Procédé de la revendication 1, et comprenant en outre l'étape d'addition d'un agent de tonicité.

16. Procédé d'une ou de plusieurs des revendications 1 à 15, comprenant en outre l'étape d'ajustement de pH de ladite solution entre 5,5 et 8.

17. Procédé de la revendication 16 et comprenant en outre l'étape d'addition d'un ingrédient actif ophtalmiquement acceptable à ladite solution qui est compatible avec lesdites traces de peroxyde d'hydrogène.

18. Procédé selon la revendication 16, et comprenant en outre l'étape d'addition de ladite solution d'un tampon et de sel physiologique en une quantité permettant de rendre la solution sensiblement isotonique.

19. Procédé de la revendication 18 dans lequel la solution a une tonicité de 0,9% de chlorure de sodium.

20. Utilisation de traces d'une source de peroxyde d'hydrogène, à l'exception de l'acide péracétique, le peroxyde d'hydrogène résultant étant stabilisé par une quantité efficace d'un stabilisateur de peroxyde d'hydrogène, comme agent de conservation pour une solution ophtalmique comprenant un principe ophtalmiquement actif ou excipient qui est compatible avec le peroxyde d'hydrogène, cette solution étant conçue pour être instillée directement dans l'oeil d'un être humain, ladite solution étant sensiblement isotonique et ayant un pH oculairement compatible, ladite quantité résultante de peroxyde d'hydrogène étant de 0,001% à 0,10% en poids, à condition que ladite solution, si elle a un pH qui dépasse 8, ne comprend pas de bicarbonate de sodium en une quantité suffisante pour décomposer le peroxyde d'hydrogène.

## Revendications (Revendications pour l'(les) Etat(s) contractant(s) suivant(s): ES, GR)

1. Procédé de conservation d'une solution ophtalmique comprenant un principe actif ophtalmique ou excipient qui est compatible avec le peroxyde d'hydrogène, cette solution étant conçue pour être instillée directement dans l'oeil d'un être humain, ladite solution étant sensiblement isotonique et ayant un pH oculairement compatible, comprenant les étapes de:
a) addition à ladite solution d'une source de peroxyde d'hydrogène, à l'exception de l'acide péracétique, pour fournir une trace efficace de peroxyde d'hydrogène résultant afin d'obtenir la capacité germicide désirée, et
b) dissolution dans ladite solution d'une quantité efficace d'au moins un stabilisateur de peroxyde d'hydrogène,
ladite quantité résultante de peroxyde d'hydrogène étant de 0,001% à 0,10% en poids, à condition que ladite solution, si elle a un pH dépassant 8, ne comprend pas de bicarbonate de sodium en une quantité suffisante pour décomposer le peroxyde d'hydrogène.

2. Procédé de préparation d'une solution ophtalmique conservée efficacement comprenant un principe actif ophtalmique ou un excipient qui est compatible avec le peroxyde d'hydrogène, cette solution étant conçue pour être instillée directement dans l'oeil d'un être humain, ladite solution étant sensiblement isotonique et ayant un pH oculairement compatible, comprenant les étapes de
a) addition à ladite solution une source de peroxyde d'hydrogène, à l'exception de l'acide péracétique, pour fournir une trace efficace de peroxyde d'hydrogène résultant afin d'obtenir la capacité germicide désirée, et
b) dissolution dans ladite solution une quantité efficace d'au moins un stabilisateur de peroxyde d'hydrogène,
ladite quantité résultante de peroxyde d'hydrogène étant de 0,001% à 0,10% en poids, à condition que ladite solution, si elle a un pH de 8, ne comprend pas de bicarbonate de sodium en une quantité suffisante pour décomposer le peroxyde d'hydrogène.

3. Procédé de la revendication 1, dans lequel ladite trace de peroxyde d'hydrogène est le seul agent de conservation utilisé.

4. Procédé de la revendication 1, dans lequel ladite source de peroxyde d'hydrogène est choisie dans le groupe constitué par le peroxyde d'hydrogène, le perborate de sodium décahydraté, le peroxyde de sodium et le peroxyde durée.

5. Procédé de la revendication 1, dans lequel ledit stabilisateur d'acide diéthylène triamine penta(méthylène phosphonique) ou l'acide 1-hydroxyéthylidène-1,1-diphosphonique ou un de leurs sels physiologiquement compatibles.

6. Procédé de la revendication 5, dans lequel ladite quantité efficace d'acide diéthylène triamine penta(methylène phosphonique) ou d'un de ses sels physiologiquement compatibles est de 0,002% à 0,03% en poids et ladite quantité efficace d'acide 1-hydroxyéthylidène-1,1-diphosphonique ou d'un de ses sels physiologiquement compatibles est de 0,005% à 0,2% en poids.

7. Procédé de la revendication 6, dans lequel ledit stabilisateur est l'acide diéthylène triamine penta(méthylène phosphonique).

8. Procédé de la revendication 6, dans lequel ledit stabilisateur est l'acide 1-hydroxyéthylidène-1,1-diphosphonique.

9. Procédé de la revendication 8, comprenant en outre l'étape d'addition d'une quantité efficace de glycérine.

10. Procédé de la revendication 9, dans lequel ladite glycérine est ajoutée en une quantité de 0,003% à 0,1% en poids.

11. Procédé de la revendication 1, dans lequel ladite quantité résultante de peroxyde d'hydrogène est de 0,001% à 0,10% en poids et ladite quantité efficace d'acide diéthylène triamine penta(méthylène phosphonique) ou d'un de ses sels physiologiquement compatibles, est de 0,002% à 0,03% en poids.

12. Procédé de la revendication 11, comprenant en outre l'étape d'addition d'une quantité efficace d'acide 1-hydroxyéthylidène-1,1-diphosphonique comme stabilisateur de peroxyde d'hydrogène.

13. Procédé de la revendication 12, comprenant en outre l'étape d'addition d'une quantité efficace de glycérine.

14. Procédé de la revendication 13, dans lequel ladite glycérine est ajoutée en une quantité de 0,005% à 0,1% en poids.

15. Procédé de la revendication 1, et comprenant en outre l'étape d'addition d'un agent de tonicité.

16. Procédé d'une ou de plusieurs des revendications 1 à 15, comprenant en outre l'étape d'ajustement de pH de ladite solution entre 5,5 et 8.

17. Procédé de la revendication 16 et comprenant en outre l'étape d'addition d'un principe actif ophtalmiquement acceptable à ladite solution qui est compatible avec lesdites traces de peroxyde d'hydrogène.

18. Procédé selon la revendication 16, et comprenant en outre l'étape d'addition de ladite solution d'un tampon et de sel physiologique en une quantité permettant de rendre la solution sensiblement isotonique.

19. Procédé de la revendication 18 dans lequel la solution a une tonicité de 0,9% de chlorure de sodium.

20. Utilisation de traces d'une source de peroxyde d'hydrogène, à l'exception de l'acide péracétique, le peroxyde d'hydrogène résultant étant stabilisé par une quantité efficace d'un stabilisateur de peroxyde d'hydrogène, comme agent de conservation pour une solution ophtalmique comprenant un principe actif ophtalmique ou un excipient qui est compatible avec le peroxyde d'hydrogène, cette solution étant conçue pour être instillée directement dans l'oeil d'un être humain, ladite solution étant sensiblement isotonique et ayant un pH oculairement compatible, ladite quantité résultante de peroxyde d'hydrogène étant de 0,001% à 0,10% en poids, à condition que ladite solution, si elle a un pH qui dépasse 8, ne comprend pas de bicarbonate de sodium en une quantité suffisante pour décomposer le peroxyde d'hydrogène.
